# EUROPEAN PATENT SPECIFICATION

(11) **EP 3 015 073 B1**
(45) Date of publication and mention of the grant of the patent: **19.10.2022**
(21) Application number: 15191749.9
(22) Date of filing: 27.10.2015
(51) Int. Cl.: A61B 8/00, A61B 8/08

(54) **ULTRASOUND IMAGING APPARATUS AND METHOD OF OPERATING THE SAME**
ULTRASCHALLBILDGEBUNGSVORRICHTUNG UND VERFAHREN ZUM BETRIEB DAVON
APPAREIL D'IMAGERIE À ULTRASONS ET PROCÉDÉ D'UTILISATION CORRESPONDANT

(30) Priority: 31.10.2014 KR 20140150638
(43) Date of publication of application: 04.05.2016
(73) Proprietor: Samsung Medison Co., Ltd., Gangwon-do 25108 (KR)
(72) Inventor: LEE, Jin-yong, Gangwon-do (KR); PARK, Sung-wook, Gangwon-do (KR); PARK, Jin-ki, Gangwon-do (KR); SONG, Joo-hyun, Gangwon-do (KR); LEE, Bong-heon, Gangwon-do (KR); CHANG, Hyuk-jae, Seoul (KR); CHUNG, Nam-sik, Seoul (KR); HONG, Geu-ru, Seoul (KR); KIM, Hyun-joo, Seoul (KR); CHO, In-jeong, Seoul (KR)
(74) Representative: Arnold & Siedsma

(56) References cited:
- EP-A1- 2 281 509
- EP-A1- 2 335 596
- EP-A1- 2 754 396
- US-A1- 2007 255 139

## Description

### BACKGROUND

### 1. Field

The present disclosure relates to ultrasound imaging apparatuses and methods of operating the same, and more particularly, to ultrasound imaging apparatuses and operation methods for displaying an image generated using ultrasound data related to an object.

### 2. Description of the Related Art

Ultrasound diagnosis apparatuses transmit ultrasound signals generated by transducers of a probe to an object and receive echo signals reflected from the object, thereby obtaining at least one image of an internal part of the object (e.g., soft tissues or blood flow). In particular, ultrasound diagnosis apparatuses are used for medical purposes including observation of the interior of an object, detection of foreign substances, and diagnosis of damage to the object. Such ultrasound diagnosis apparatuses provide high stability, display images in real time, and are safe due to the lack of radioactive exposure, compared to X-ray apparatuses. Therefore, ultrasound diagnosis apparatuses are widely used together with other image diagnosis apparatuses including a computed tomography (CT) apparatus, a magnetic resonance imaging (MRI) apparatus, and the like.

US 2007/0255139 A1 discloses an ultrasound imaging apparatus comprising the features of the preamble of claim 1.

EP-2754396 and EP-2335596 are acknowledged as prior art and disclose generating an ultrasound reference image of an arbitrary cross-section by using 3D volume data, and selecting a 2D slice image from a 3D ultrasound image, respectively.

### SUMMARY

Provided are ultrasound imaging apparatuses and methods of operating the same, which are capable of providing, by using ultrasound data, an ultrasound image of a cross-section that a user desires to observe.

Additional aspects will be set forth in part in the description which follows and, in part, will be apparent from the description, or may be learned by practice of the presented exemplary embodiments.

According to a first aspect of the present invention, an ultrasound imaging apparatus comprises the features defined in the appended independent apparatus claim

According to a second aspect of the present invention, a method of operating an ultrasound imaging apparatus comprises at least all features defined in the appended independent method claim.

Preferred embodiments are defined in the dependent claims.

### BRIEF DESCRIPTION OF THE DRAWINGS

Exemplary embodiments will now be described more fully hereinafter with reference to the accompanying drawings in which reference numerals denote structural elements:
FIG. 1 is a block diagram of a configuration of an ultrasound diagnosis apparatus according to an exemplary embodiment;
FIG. 2 is a block diagram of a configuration of a wireless probe according to an exemplary embodiment;
FIG. 3 is a block diagram of an ultrasound imaging apparatus according to an exemplary embodiment;
FIG. 4 is block diagram of an ultrasound imaging apparatus according to another exemplary embodiment;
FIG. 5 is a diagram for explaining a user interface screen for selecting a reference ultrasound image via an ultrasound imaging apparatus, according to an exemplary embodiment;
FIGS. 6 and 7 are diagrams for explaining user interface screens for setting a user-designated reference cross-section, according to an exemplary embodiment;
FIGS. 8 through 11 are diagrams for explaining screens on which a first ultrasound image corresponding to a user-designated reference cross-section is displayed, according to an exemplary embodiment; and
FIG. 12 is a flowchart of a method of operating an ultrasound imaging apparatus, according to an exemplary embodiment.

### DETAILED DESCRIPTION

The terms used in this specification are those general terms currently widely used in the art. Also, some terms may be arbitrarily selected by the applicant, and in this case, the meaning of the selected terms will be described in detail in the detailed description of the present specification. Thus, the terms used herein have to be defined based on the meaning of the terms together with the description throughout the specification.

Hereinafter, the terms used in the specification will be briefly described, and then the present invention will be described in detail.

When a part "includes" or "comprises" an element, unless there is a particular description contrary thereto, the part can further include other elements, not excluding the other elements. Also, the term "unit" in the embodiments of the present invention means a software component or hardware components such as a field-programmable gate array (FPGA) or an application-specific integrated circuit (ASIC), and performs a specific function. However, the term "unit" is not limited to software or hardware. The "unit" may be formed so as to be in an addressable storage medium, or may be formed so as to operate one or more processors. Thus, for example, the term "unit" may refer to components such as software components, object-oriented software components, class components, and task components, and may include processes, functions, attributes, procedures, subroutines, segments of program code, drivers, firmware, micro codes, circuits, data, a database, data structures, tables, arrays, or variables. A function provided by the components and "units" may be associated with the smaller number of components and "units", or may be divided into additional components and "units".

It will be understood that, although the terms "first", "second", etc. may be used herein to describe various elements, these elements should not be limited by these terms. These terms are only used to distinguish one element from another element. Thus, a first element discussed below could be termed a second element, and similarly, a second element could be termed a first element, without departing from the scope of example embodiments. As used herein, the term "and/or" includes any and all combinations of one or more of the associated listed items. Expressions such as "at least one of," when preceding a list of elements, modify the entire list of elements and do not modify the individual elements of the list.

Throughout the specification, an "image" may refer to multi-dimensional data composed of discrete image elements (e.g., pixels in a two-dimensional (2D) image and voxels in a three-dimensional (3D) image).

Throughout the specification, an "ultrasound image" refers to an image of an object, which is obtained using ultrasound waves. Ultrasound imaging apparatuses transmit ultrasound signals generated by transducers of a probe to an object and receive echo signals reflected from the object, thereby obtaining at least one image of an internal part of the object.

Furthermore, an "object" may be a human, an animal, or a part of a human or animal. For example, the object may be an organ (e.g., the liver, the heart, the womb, the brain, a breast, or the abdomen), a blood vessel, or a combination thereof. Also, the object may be a phantom. The phantom means a material having a density, an effective atomic number, and a volume that are approximately the same as those of an organism. For example, the phantom may be a spherical phantom having properties similar to a human body.

Furthermore, throughout the specification, a "user" may be, but is not limited to, a medical expert, such as a medical doctor, a nurse, a medical laboratory technologist, a medical image expert, or a technician who repairs a medical apparatus.

Embodiments of the invention now will be described more fully hereinafter with reference to the accompanying drawings, in which illustrative embodiments of the invention are shown.

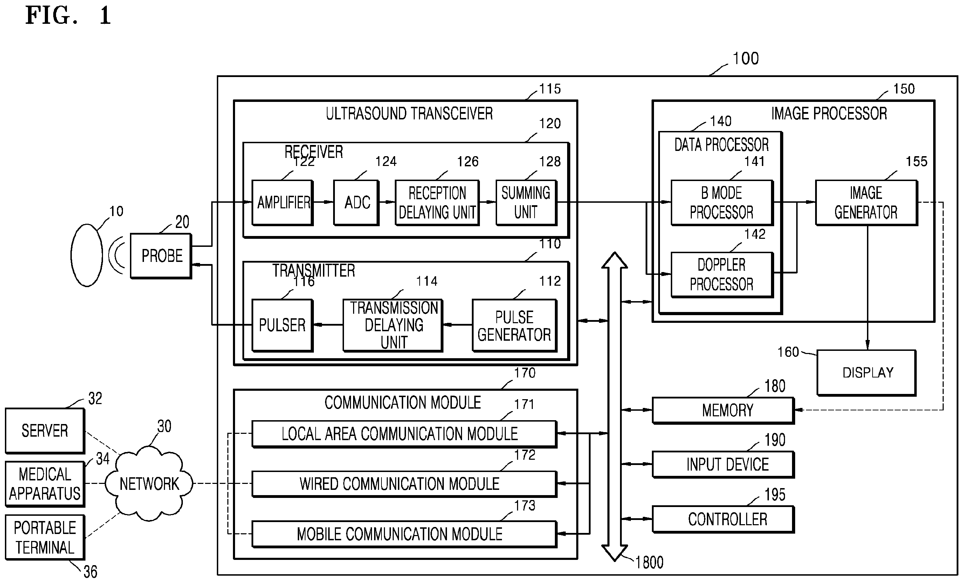

FIG. 1 is a block diagram showing a configuration of an ultrasound diagnosis apparatus 100 according to an embodiment.

Referring to FIG. 1, the ultrasound diagnosis apparatus 100 according to the present exemplary embodiment may include a probe 20, an ultrasound transceiver 115, an image processor 150, a display 160, a communication module 170, a memory 180, an input device 190 and a controller 195, which may be connected to one another via buses 185. The image processor 150 may include an image generator 155, a section information detector 130, and the display 160.

It will be understood by those of ordinary skill in the art that the ultrasound diagnosis apparatus 100 may further include common components other than those illustrated in FIG. 1.

In some embodiments, the ultrasound diagnosis apparatus 100 may be a cart type apparatus or a portable type apparatus. Examples of portable ultrasound diagnosis apparatuses may include, but are not limited to, a picture archiving and communication system (PACS) viewer, a smartphone, a laptop computer, a personal digital assistant (PDA), and a tablet PC.

The probe 20 transmits ultrasound waves to an object 10 in response to a driving signal applied by the ultrasound transceiver 115 and receives echo signals reflected by the object 10. The probe 20 includes a plurality of transducers, and the plurality of transducers oscillate in response to electric signals and generate acoustic energy, that is, ultrasound waves. Furthermore, the probe 20 may be connected to the main body of the ultrasound diagnosis apparatus 100 by wire or wirelessly, and according to embodiments, the ultrasound diagnosis apparatus 100 may include a plurality of probes 20.

A transmitter 110 supplies a driving signal to the probe 20. The transmitter 110 includes a pulse generator 112, a transmission delaying unit 114, and a pulser 116. The pulse generator 112 generates pulses for forming transmission ultrasound waves based on a predetermined pulse repetition frequency (PRF), and the transmission delaying unit 114 delays the pulses by delay times necessary for determining transmission directionality. The pulses which have been delayed correspond to a plurality of piezoelectric vibrators included in the probe 20, respectively. The pulser 116 applies a driving signal (or a driving pulse) to the probe 20 based on timing corresponding to each of the pulses which have been delayed.

A receiver 120 generates ultrasound data by processing echo signals received from the probe 20. The receiver 120 may include an amplifier 122, an analog-to-digital converter (ADC) 124, a reception delaying unit 126, and a summing unit 128. The amplifier 122 amplifies echo signals in each channel, and the ADC 124 performs analog-to-digital conversion with respect to the amplified echo signals. The reception delaying unit 126 delays digital echo signals output by the ADC 1124 by delay times necessary for determining reception directionality, and the summing unit 128 generates ultrasound data by summing the echo signals processed by the reception delaying unit 1126.

The image processor 150 generates an ultrasound image by scan-converting ultrasound data generated by the ultrasound transceiver 115.

According to an embodiment, the image generator 155 may generate a three-dimensional (3D) ultrasound image via volume-rendering with respect to volume data and may also generate an elasticity image by imaging deformation of the object 10 due to pressure. Furthermore, the image generator 155 may display various pieces of additional information in an ultrasound image by using text and graphics. In addition, the generated ultrasound image may be stored in the memory 180.

A display 160 displays the generated ultrasound image. The display 160 may display not only an ultrasound image, but also various pieces of information processed by the ultrasound diagnosis apparatus 100 on a screen image via a graphical user interface (GUI). In addition, the ultrasound diagnosis apparatus 100 may include two or more displays 160 according to embodiments.

The display 160 may include at least one of a liquid crystal display (LCD), a thin film transistor-LCD (TFT-LCD), an organic light-emitting diode (OLED) display, a flexible display, a 3D display, and an electrophoretic display.

Furthermore, when the display 160 and the input device 190 form a layer structure to form a touch screen, the display 160 may be used as an input device as well as an output device, via which a user inputs information via a touch.

The touch screen may be configured to detect a position of a touch input, a touched area, and pressure of a touch. The touch screen may also be configured to detect both a real touch and a proximity touch.

In the present specification, a 'real touch' means that a pointer actually touches a screen, and a 'proximity touch' means that a pointer does not actually touch a screen but approaches the screen while being separated from the screen by a predetermined distance. A 'pointer' used herein means a tool for touching a particular portion on or near a displayed screen. Examples of the pointer may include a stylus pen and a body part such as a finger.

Although not shown, the ultrasound diagnosis apparatus 100 may include various sensors that are disposed within or near the touch screen so as to sense a real touch or proximity touch on the touch screen. A tactile sensor is an example of the sensors for sensing a touch on the touch screen.

The tactile sensor is used to sense a touch of a particular object to the same or greater degree than the degree to which a human can sense the touch. The tactile sensor may detect various pieces of information including the roughness of a contact surface, the hardness of an object to be touched, the temperature of a point to be touched, etc.

A proximity sensor is another example of the sensors for sensing a touch. The proximity sensor refers to a sensor that senses the presence of an object that is approaching or is located near a predetermined detection surface by using the force of an electromagnetic field or infrared light without any mechanical contact.

Examples of the proximity sensor include a transmissive photoelectric sensor, a direct reflective photoelectric sensor, a mirror reflective photoelectric sensor, a high-frequency oscillation proximity sensor, a capacitive proximity sensor, a magnetic proximity sensor, an infrared proximity sensor, and the like.

The communication module 170 is connected to a network 30 by wire or wirelessly to communicate with an external device or a server. The communication module 170 may exchange data with a hospital server or another medical apparatus in a hospital, which is connected thereto via a PACS. Furthermore, the communication module 170 may perform data communication according to the digital imaging and communications in medicine (DICOM) standard.

The communication module 170 may transmit or receive data related to diagnosis of an object, e.g., an ultrasound image, ultrasound data, and Doppler data of the object, via the network 30 and may also transmit or receive medical images captured by another medical apparatus, e.g., a computed tomography (CT) apparatus, a magnetic resonance imaging (MRI) apparatus, or an X-ray apparatus. Furthermore, the communication module 170 may receive information about a diagnosis history or medical treatment schedule of a patient from a server and utilizes the received information to diagnose the patient. Furthermore, the communication module 170 may perform data communication not only with a server or a medical apparatus in a hospital, but also with a portable terminal of a medical doctor or patient.

The communication module 170 is connected to the network 30 by wire or wirelessly to exchange data with a server 32, a medical apparatus 34, or a portable terminal 36. The communication module 170 may include one or more components for communication with external devices. For example, the communication module 1300 may include a local area communication module 171, a wired communication module 172, and a mobile communication module 173.

The local area communication module 171 refers to a module for local area communication within a predetermined distance. Examples of local area communication techniques according to an embodiment may include, but are not limited to, wireless LAN, Wi-Fi, Bluetooth, ZigBee, Wi-Fi Direct (WFD), ultra wideband (UWB), infrared data association (IrDA), Bluetooth low energy (BLE), and near field communication (NFC).

The wired communication module 172 refers to a module for communication using electric signals or optical signals. Examples of wired communication techniques according to an embodiment may include communication via a twisted pair cable, a coaxial cable, an optical fiber cable, and an Ethernet cable.

The mobile communication module 173 transmits or receives wireless signals to or from at least one selected from a base station, an external terminal, and a server on a mobile communication network. The wireless signals may be voice call signals, video call signals, or various types of data for transmission and reception of text/multimedia messages.

The memory 180 stores various data processed by the ultrasound diagnosis apparatus 100. For example, the memory 180 may store medical data related to diagnosis of an object, such as ultrasound data and an ultrasound image that are input or output, and may also store algorithms or programs which are to be executed in the ultrasound diagnosis apparatus 100.

The memory 180 may be any of various storage media, e.g., a flash memory, a hard disk drive, EEPROM, etc. Furthermore, the ultrasound diagnosis apparatus 100 may utilize web storage or a cloud server that performs the storage function of the memory 180 online.

The input device 190 generates input data that the user inputs for controlling an operation of the ultrasound diagnosis apparatus 100. The user input 190 may include hardware components, such as a keypad, a mouse, a touch pad, a track ball, and a jog switch.

In particular, the input device 190 may also include a touch screen in which a touch pad forms a layer structure with the display 160.

In this case, according to an exemplary embodiment, the ultrasound diagnosis apparatus 100 may display an ultrasound image in a predetermined mode and a control panel for the ultrasound image on a touch screen. The ultrasound diagnosis apparatus 100 may also sense a user's touch gesture performed on an ultrasound image via a touch screen.

According to an exemplary embodiment, the ultrasound diagnosis apparatus 100 may include some buttons that are frequently used by a user among buttons that are included in a control panel of a general ultrasound apparatus, and provide the remaining buttons in the form of a graphical user interface (GUI) via a touch screen.

The controller 195 may control all operations of the ultrasound diagnosis apparatus 100. In other words, the controller 195 may control operations among the probe 20, the ultrasound transceiver 100, the image processor 150, the communication module 170, the memory 180, and the user input 190 shown in FIG. 1.

All or some of the probe 20, the ultrasound transceiver 115, the image processor 150, the communication module 170, the memory 180, the user input 190, and the controller 195 may be implemented as software modules. However, embodiments of the present invention are not limited thereto, and some of the components stated above may be implemented as hardware modules. Also, at least one of the ultrasound transmission/reception unit 115, the image processor 150, and the communication module 170 may be included in the control unit 195.

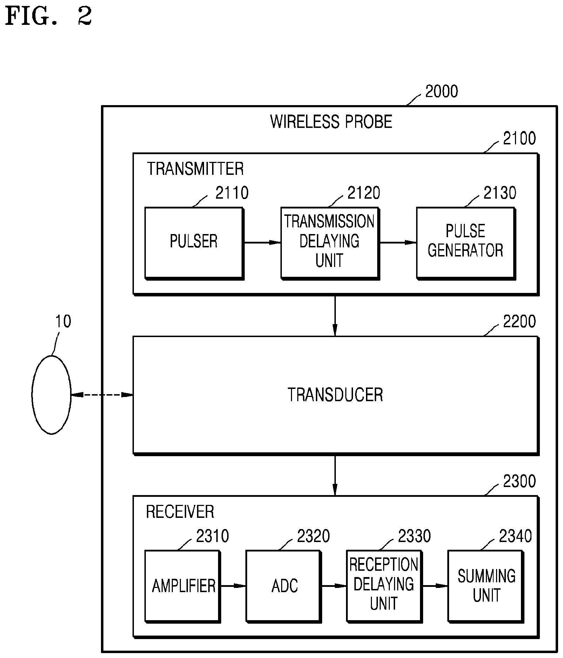

FIG. 2 is a block diagram showing a configuration of a wireless probe 2000 according to an embodiment. As described above with reference to FIG. 1, the wireless probe 2000 may include a plurality of transducers, and, according to embodiments, may include some or all of the components of the ultrasound transceiver 100 shown in FIG. 1.

The wireless probe 2000 according to the embodiment shown in FIG. 2 includes a transmitter 2100, a transducer 2200, and a receiver 2300. Since descriptions thereof are given above with reference to FIG. 1, detailed descriptions thereof will be omitted here. In addition, according to embodiments, the wireless probe 2000 may selectively include a reception delaying unit 2330 and a summing unit 2340.

The wireless probe 2000 may transmit ultrasound signals to the object 10, receive echo signals from the object 10, generate ultrasound data, and wirelessly transmit the ultrasound data to the ultrasound diagnosis apparatus 1000 shown in FIG. 1.

The wireless probe 2000 may be a smart device including a transducer array that is capable of performing an ultrasound scan. In detail, the wireless probe 2000 is a smart device that acquires ultrasound data by scanning an object via the transducer array. Then, the wireless probe 2000 may generate an ultrasound image by using the acquired ultrasound data and/or display the ultrasound image. The wireless probe 2000 may include a display (not shown) via which a screen including at least one ultrasound image and/or a user interface screen for controlling an operation of scanning an object may be displayed.

While the user is scanning a predetermined body part of a patient that is an object by using the wireless probe 2000, the wireless probe 2000 and the ultrasound diagnosis apparatus 100 may continue to transmit or receive certain data therebetween via a wireless network. In detail, while the user is scanning a predetermined body part of a patient that is an object by using the wireless probe 2000, the wireless probe 2000 may transmit ultrasound data to the ultrasound diagnosis apparatus 100 in real-time via the wireless network. The ultrasound data may be updated in real-time as an ultrasound scan continues and then be transmitted from the wireless probe 2000 to the ultrasound diagnosis apparatus 100.

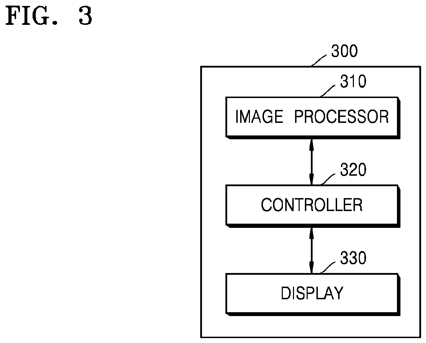

FIG. 3 is a block diagram of a configuration of an ultrasound imaging apparatus 300 according to an exemplary embodiment.

Referring to FIG. 3, the ultrasound imaging apparatus 300 according to the present exemplary embodiment may include an image processor 310, a controller 320, and a display 330. However, the ultrasound imaging apparatus 300 may include more components than those shown in FIG. 3.

Since the image processor 310, the controller 320, and the display 330 of the ultrasound imaging apparatus 300 of FIG. 3 respectively correspond to the image processor 150, the controller 195, and the display 160 of the ultrasound diagnosis apparatus 100 of FIG. 1, descriptions already provided with respect to FIG. 1 will be omitted below.

The image processor 310 is configured to acquire, based on 3D ultrasound data for generating a 3D ultrasound image of an object 10, at least one 2D ultrasound image including at least one reference ultrasound image corresponding to at least one reference cross-section.

The controller 320 is configured to set a user-designated reference cross-section obtained by moving a first reference cross-section included in the at least one reference cross-section and control generation of at least one first ultrasound image corresponding to the user-designated reference cross-section.

The display 330 is configured to display at least one first ultrasound image.

The image processor 310 is configured to acquire at least one 2D ultrasound image based on 3D ultrasound data for generating a 3D ultrasound image of the object 10. A 2D ultrasound image may be an image corresponding to a cross-section that is perpendicular to a first axis in a 3D ultrasound image, a cross-section including the first axis, and a cross-section that is parallel to the first axis.

The object 10 may be the heart. The image processor 310 may acquire, based on 3D ultrasound data related to the heart, at least one 2D ultrasound image corresponding to at least one cross-section of the heart and being used to analyze the heart. In this case, the at least one 2D ultrasound image may be a cross-sectional image obtained along at least one of long and short axes. A cross-sectional image obtained along a long axis of the heart may be a cross-sectional image provided for a 2-chamber view, a 3-chamber view, a 4-chamber view, etc. in with respect to the long axis. Furthermore, a cross-sectional image obtained along a short axis of the heart may be a cross-sectional image provided for apical, mid, and basal views, etc., in with respect to the short axis.

In an embodiment not forming part of the present invention, the image processor 310 may generate at least one 2D ultrasound image based on 3D ultrasound data related to the object 10 and receive at least one previously generated 2D ultrasound image. In this case, the ultrasound imaging apparatus 300 may receive at least one 2D ultrasound image from an external device that is physically independent of the ultrasound imaging apparatus 300. The external device may be an ultrasound diagnosis apparatus for acquiring a 2D ultrasound image by using 3D ultrasound data related to the object 10 or a storage device for storing a 2D ultrasound image.

At least one of the acquired at least one 2D ultrasound image is determined as a reference ultrasound image. The reference ultrasound image corresponds to a reference cross-section in a 3D ultrasound image. Each of a plurality of reference ultrasound images has a corresponding reference cross-section in a 3D ultrasound image.

The controller 320 is configured to control the image processor 310 to generate at least one first ultrasound image that is distinguished from the at least one 2D ultrasound image. The controller 320 may set a user-designated reference cross-section. The user-designated reference cross-section is set for a user to analyze an image from a different viewpoint than that of the at least one 2D ultrasound image. The user-designated reference cross-section is obtained by moving a first reference cross-section corresponding to the at least one 2D ultrasound image. For example, the user-designated reference cross-section may be obtained by rotating the first reference cross-section by 30 degrees clockwise. Furthermore, the user-designated reference cross-section may be obtained by moving the first reference cross-section by a certain distance in a horizontal direction, but is not limited thereto. The controller 320 controls the image processor 310 to generate at least one first ultrasound image corresponding to the user-designated reference cross-section.

The controller 320 controls the display 330 to display a predetermined screen. The display 330 may display the predetermined screen so that a user or patient may visually recognize a predetermined image or information. The display 330 may correspond to the display 160 shown in FIG. 1 or be separate from the ultrasound diagnosis apparatus 100 of FIG. 1.

The display 330 may display a predetermined screen. In detail, the display 330 may display the predetermined screen according to control by the controller 320. The display 330 includes a display panel (not shown) and displays a user interface screen, a medical image screen, etc. on the display panel.

The display 330 is configured to display the generated at least one first ultrasound image. Furthermore, the display 330 may display a screen including at least one of the at least one 2D ultrasound image and a 3D ultrasound image. Furthermore, the display 330 may display a screen including the at least one of at least one 2D ultrasound image and the 3D ultrasound image, as well as the at least one first ultrasound image.

The display 330 may display each of a user-designated reference cross-section and a first reference cross-section corresponding to a reference ultrasound image in such a manner as to overlap a 3D ultrasound image. In other words, the display 330 may display a screen where the first reference cross-section and the user-designated reference cross-section are respectively located on corresponding cross-sections in the 3D ultrasound image. The display 330 may generate marks for the first reference cross-section and the user-designated reference cross-section that are respectively located at corresponding positions in the 3D ultrasound image and display the marks respectively corresponding to the first reference cross-section and the user-designated reference cross-section. Thus, the user may identify a cross-section in a 3D image corresponding to a view for observing a 2D image.

The display 330 may display a screen including at least one of a 3D ultrasound image, at least one 2D ultrasound image, and at least one first ultrasound image.

The display 330 is configured to display a screen including at least one of at least one 2D ultrasound image and at least one of at least one first ultrasound image. The at least one 2D ultrasound image and the at least one first ultrasound image are displayed in such a manner as to be distinguished from each other. For example, edges of 2D and 3D ultrasound images may be respectively processed and displayed as a solid line and a dashed line.

The display 330 may display a 3D ultrasound image including a first reference cross-section and at least one 2D ultrasound image in such a manner that they are associated with each other. Furthermore, the display 330 may display a 3D ultrasound image including a user-designated reference cross-section and at least one first ultrasound image in such a manner that they are associated with each other. For example, the display 330 may display on a single screen a 2D ultrasound image and a 3D ultrasound image on which a first reference cross-section corresponding to the 2D ultrasound image is marked. Furthermore, the display 330 may display on a single screen a first ultrasound image and a 3D ultrasound image on which a user-designated reference cross-section corresponding to the first ultrasound image is marked.

In accordance with the invention, the image processor 310 is configured to receive 3D ultrasound data that is different from previously generated 3D ultrasound data. In this case, the different 3D ultrasound data may be acquired by the ultrasound imaging apparatus 300 or be received from an external device. The external device is a device for acquiring, storing, processing, or using data related to an ultrasound image, and may be a medical imaging apparatus, a medical server, a portable terminal, or any other computing device for using and processing a medical image. For example, the external device may be a medical diagnosis apparatus included in a medical institution such as a hospital. Furthermore, the external device may be a server in a hospital for recording and storing a patient's clinical history, a medical imaging apparatus used by a medical doctor in a hospital to read a medical image, or the like.

The controller 320 is configured to control the image processor 310 to generate at least one second ultrasound image corresponding to a user-designated reference cross-section by using 3D ultrasound data that is different from the existing 3D ultrasound data. In this case, the controller 320 is configured to control the image processor 310 to acquire the at least one second ultrasound image by using a preset user-designated reference cross-section without having to reset the user-designated reference cross-section. Thus, the ultrasound imaging apparatus 300 provides the 2D ultrasound image corresponding to the user-designated reference cross-section within a short time. The display 330 is configured to display the generated at least one second ultrasound image.

In addition, the image processor 310 generates a window and acquires a first ultrasound image corresponding to a user-designated reference cross-section. In detail, the image processor 310 generates a window to be located on a 3D ultrasound image. The image processor 310 may move the window to a position corresponding to the user-designated reference cross-section in the 3D ultrasound image by using setting information of the user-designated reference cross-section. The image processor 310 may acquire at least one first ultrasound image at the position corresponding to the user-designated reference cross-section.

The ultrasound imaging apparatus 300 may include a central arithmetic processor that controls overall operations of the image processor 310, the controller 320, and the display 330. The central arithmetic processor may be implemented as an array of a plurality of logic gates or a combination of a general purpose microprocessor and a program that can be run on the general purpose microprocessor. Furthermore, it will be appreciated by those of ordinary skill in the art to which the present embodiment pertains that the central arithmetic processor may be formed using different types of hardware

Hereinafter, various operations performed by the ultrasound imaging apparatus 300 and applications thereof will be described in detail. Although none of the image processor 310, the controller 320, and the display 330 are specified, features and aspects that would be clearly understood by and are obvious to those of ordinary skill in the art may be considered as a typical implementation. The scope of the present inventive concept is not limited by a name of a particular component or physical/logical structure.

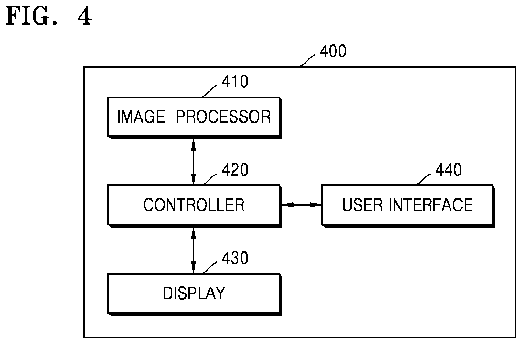

FIG. 4 is a block diagram of a configuration of an ultrasound imaging apparatus 400 according to another exemplary embodiment. Unlike the ultrasound imaging apparatus 300 of FIG. 3, the ultrasound imaging apparatus 400 according to the present exemplary embodiment may further include a user interface 440.

Since an image processor 410, a controller 420, and a display 430 of the ultrasound imaging apparatus 400 of FIG. 4 respectively correspond to the image processor 310, the controller 320, and the display 330 of the ultrasound imaging apparatus 300 of FIG. 3, descriptions already provided with respect to FIG. 3 will be omitted below.

The user interface 440 may receive a first user input for moving a first reference cross-section. In this case, the first user input includes at least one of an input for selecting a first reference ultrasound image corresponding to the first reference cross-section from among at least one 2D ultrasound image and an input of setting information for setting the user-designated reference cross-section. The user-designated reference cross-section may be set to move the first reference cross-section. The setting information may include at least one of rotation information, tilting information, and vertical movement information that are set with respect to the first reference cross-section.

The user interface 440 refers to a device via which data for controlling the ultrasound imaging apparatus 400 is received from a user. The user interface 440 may include hardware components, such as a keypad, a mouse, a touch panel, a touch screen, a track ball, and a jog switch, but is not limited thereto. The user interface 440 may further include any of various other input units including an electrocardiogram (ECG) measuring module, a respiration measuring module, a voice recognition sensor, a gesture recognition sensor, a fingerprint recognition sensor, an iris recognition sensor, a depth sensor, a distance sensor, etc.

The user interface 440 may generate and output a user interface screen for receiving a predetermined command or data from the user. The user interface 440 may also receive the predetermined command or data from the user via the user interface screen. The user may view the user interface screen displayed via the display 430 to visually recognize predetermined information and input a predetermined command or data via the user interface 440.

For example, the user interface 440 may be formed as a touch pad. In detail, the user interface 440 includes a touch pad combined with the display panel in the display 430. In this case, a user interface screen is output to the display panel. When a predetermined command is input via the user interface screen, the touch pad may detect information about the predetermined command and then transmit the detected information to the controller 420. Then, the controller 420 may interpret the detected information to recognize and execute the predetermined command input by the user.

The ultrasound imaging apparatus 400 may further include a storage device (not shown) and a communication module (not shown). The storage device may store data related to an object (e.g., an ultrasound image, ultrasound data, scan-related data, data related to diagnosis of a patient, etc.), data transmitted from an external device to the ultrasound imaging apparatus 400. The data transmitted from the external device may include patient-related information, data necessary for diagnosis and treatment of a patient, a patient's past medical history, a medical work list corresponding to instructions regarding diagnosis of a patient, and the like.

The communication module may receive and/or transmit data from and/or to an external device. For example, the communication module may connect to a wireless probe or an external device via a communication network based on Wi-Fi or Wi-Fi Direct (WFD) technology. In detail, examples of a wireless communication network to which the communication module can connect may include, but are not limited to, Wireless LAN (WLAN), Wi-Fi, Bluetooth, ZigBee, WFD, Ultra Wideband (UWB), Infrared Data Association (IrDA), Bluetooth Low Energy (BLE), and Near Field Communication (NFC).

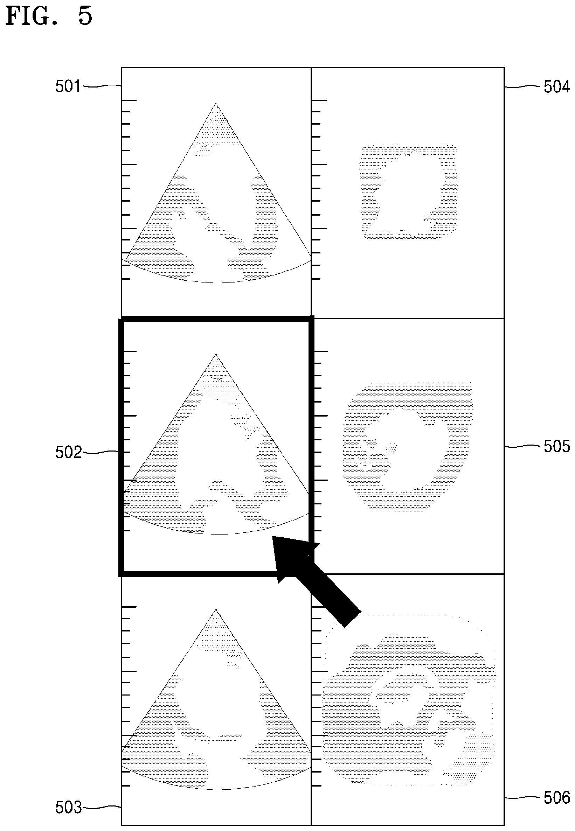

FIG. 5 is a diagram for explaining a user interface screen for selecting a reference ultrasound image via the ultrasound imaging apparatus 300, according to an exemplary embodiment.

Referring to FIG. 5, the ultrasound imaging apparatus 300 may display a screen 500 on which a plurality of 2D ultrasound images of an object are displayed. In detail, the ultrasound imaging apparatus 300 may display a plurality of 2D ultrasound images obtained by capturing ultrasound images along long and short axes of the heart. When the object is the heart, FIG. 5 shows a screen including a plurality of 2D ultrasound images of the heart.

In order to check for the presence of heart disease, the user may detect and diagnose a suspected part of the heart by acquiring ultrasound images along multiple axes of the heart and analyzing the acquired ultrasound images. To identify the presence of heart disease, ultrasound images showing an apex and a base of the heart are needed.

The ultrasound imaging apparatus 300 may display, based on a long axis of the heart, cross-sectional images 501 through 503 respectively for 2-chamber, 3-chamber, and 4-chamber views by using cross-sections including the long axis. Furthermore, the ultrasound imaging apparatus 300 may display, based on a short axis of the heart, cross-sectional images 504 through 506 respectively provided for apical, mid, and basal views by using cross-sections that is perpendicular to the short axis. Such a plurality of cross-sectional images may be arranged in a manner predesignated by the user and output via a display panel. While cross-sectional images provided by a general ultrasound imaging apparatus are designated and do not satisfy the user's intention, images provided by the ultrasound imaging apparatus 300 may be composed based on a cross-section that the user desires to observe. Thus, the ultrasound imaging apparatus 300 may provide ultrasound images of the object 10 more efficiently from multiple viewpoints than general ultrasound imaging apparatuses.

The ultrasound imaging apparatus 300 may display a screen including 2D ultrasound images obtained along long and short axes of the heart. The ultrasound imaging apparatus 300 may output a screen including a plurality of 2D ultrasound images as a user interface screen. The user interface screen may be formed as a touch pad. In this case, when an input for selecting a reference ultrasound image is performed via the user interface screen, the touch pad detects the input and transmits the detected input to an image processor and a controller. For example, the ultrasound imaging apparatus 300 may receive an input for selecting one of the plurality of 2D ultrasound images as a reference ultrasound image. The ultrasound imaging apparatus 300 performs a process for setting a user-designated reference cross-section based on the selected reference ultrasound image.

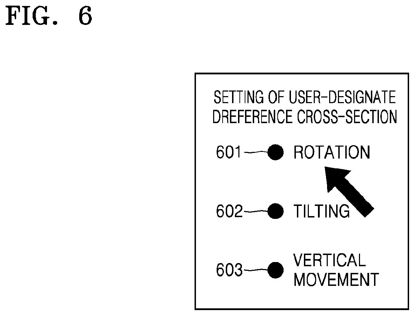

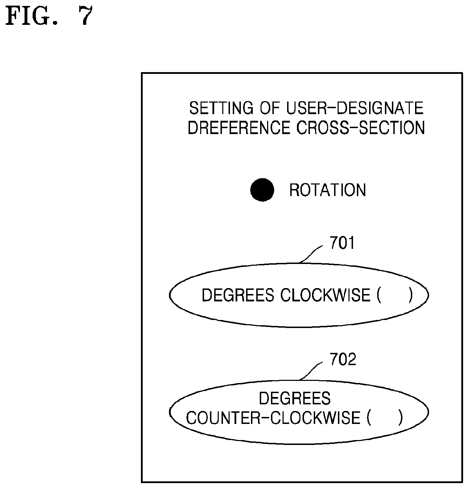

FIGS. 6 and 7 are diagrams for explaining a user interface screen for setting a user-designated reference cross-section.

Referring to FIG. 6, when the ultrasound imaging apparatus 300 receives a signal for selecting a reference ultrasound image, the ultrasound imaging apparatus 300 displays a screen for setting a user-designated reference cross-section. Since the reference ultrasound image is a 2D ultrasound image, the reference ultrasound image has a corresponding reference cross-section for acquiring the 2D ultrasound image. Furthermore, the reference cross-section is a cross-section in a 3D ultrasound image, which is used to observe a view of the 2D ultrasound image.

The ultrasound imaging apparatus 300 may receive a signal for selecting at least one reference ultrasound image from among a plurality of 2D ultrasound images. Each of the at least one reference ultrasound image has a corresponding reference cross-section in a 3D ultrasound image.

A user-designated reference cross-section is obtained by moving a first reference cross-section included in the at least one reference cross-section. The ultrasound imaging apparatus 300 may set the user-designated reference cross-section by using an angle or distance relative to the first reference cross-section. The ultrasound imaging apparatus 300 may receive setting information for moving the first reference cross-section. In this case, the setting information may include at least one of rotation information, tilting information, and vertical movement information that are set with respect to the first reference cross-section.

In detail, the ultrasound imaging apparatus 300 displays a screen for selecting parameters 601 through 603 used for moving the first reference cross-section. For example, the parameters 601 through 603 may include a rotation parameter 601 indicating the amount by which the first reference cross-section rotates, a tilting parameter 602 indicating the extent to which the first reference cross-section is tilted, and a vertical movement parameter 603 indicating the amount of vertical movement of the first reference cross-section. Those of ordinary skill in the art to which the present embodiment pertains will understand that the user-designated reference cross-section may be set using parameters other than the above-described parameters 601 through 603.

Furthermore, the ultrasound imaging apparatus 300 may set the user-designated reference cross-section by combining values for at least one parameter from among the rotation parameter 601, the tilting parameter 602, and the vertical movement parameter 603.

The ultrasound imaging apparatus 300 may receive a signal for selecting at least one of the parameters 601 through 603 used for setting the user-designated reference cross-section. Referring to FIG. 6, the ultrasound imaging apparatus 300 may receive a signal for selecting the rotation parameter 601.

Referring to FIG. 7, the ultrasound imaging apparatus 300 displays a screen for inputting information about the amount of degrees by which and the direction in which the first reference cross-section rotates, according to a result of selecting the rotation parameter 601. The user may input values 701 and 702 for the rotation parameter 601 via a user interface. The user may set a plurality of user-designated reference cross-sections in a 3D ultrasound image in order to generate views of 2D ultrasound images that the user desires to observe. The user may input via the user interface a parameter and a value of the parameter for each of the plurality of user-designated reference cross-sections.

The ultrasound imaging apparatus 300 may store in a storage device (not shown) a parameter used for setting a user-designated reference cross-section and a value of the parameter. Furthermore, the storage device may be built into the ultrasound imaging apparatus 300 or be implemented in an external device that is physically independent of the ultrasound imaging apparatus 300. The storage device may be any of various storage media such as a hard disk drive (HDD), Read Only Memory (ROM), Random Access Memory (RAM), a flash memory, and a memory card.

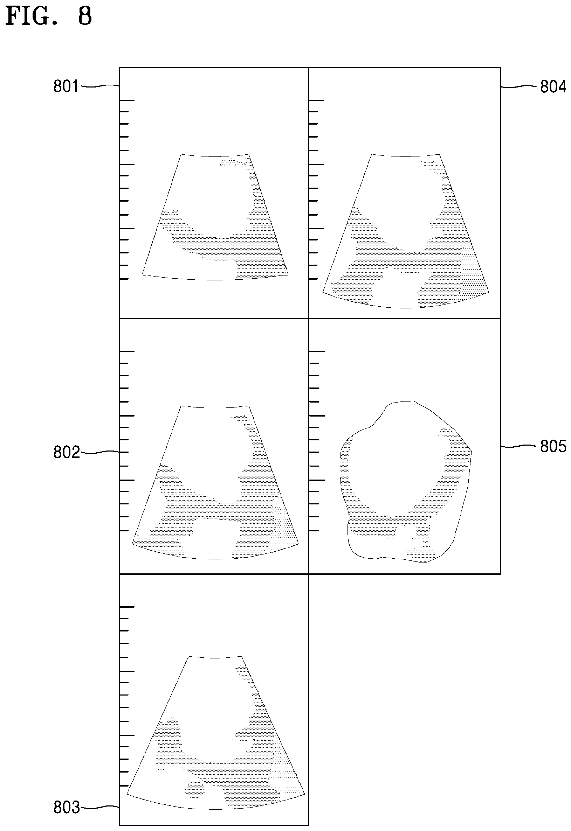

FIGS. 8 through 11 are diagrams for explaining screens on which a first ultrasound image corresponding to a user-designated reference cross-section is displayed, according to an exemplary embodiment.

Referring to FIG. 8, the ultrasound imaging apparatus 300 displays a screen on which a plurality of first ultrasound images 801 through 805 respectively corresponding to a plurality of user-designated reference cross-sections are displayed. When a signal for selecting one image from among the plurality of first ultrasound images 801 through 805 is received, the ultrasound imaging apparatus 300 may display the selected one image.

As shown in FIG. 8, the first ultrasound images 801 through 805 may be 2D cross-sectional images obtained along a long axis of the heart. The ultrasound imaging apparatus 300 may display the plurality of first ultrasound images 801 through 805 respectively corresponding to the user-designated reference cross-sections and arranged in an order designated by the user.

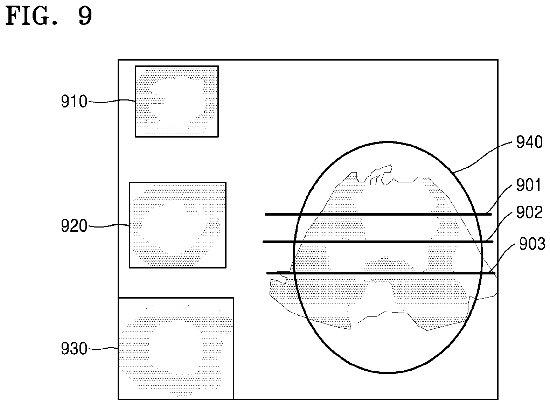

Referring to FIG. 9, when an object is the heart, the ultrasound imaging apparatus 300 may display a screen on which first ultrasound images 910, 920, and 930 respectively corresponding to user-designated reference cross-sections 901 through 903 are displayed together with a 3D ultrasound image 940. The ultrasound imaging apparatus 300 displays the first ultrasound images 910, 920, and 930 respectively corresponding to the user-designated reference cross-sections 901 through 903 in the 3D ultrasound image 940, which are set based on a first reference cross-section with respect to a short axis of the heart. Furthermore, the ultrasound imaging apparatus 300 indicates positions of the user-designated reference cross-sections 901 through 903 respectively corresponding to the first ultrasound images 910, 920, and 930.

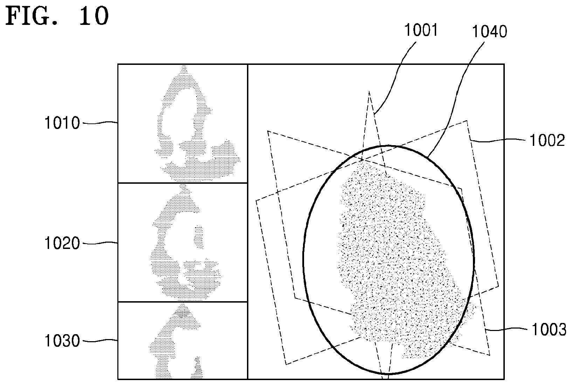

Referring to FIG. 10, when the object is the heart, the ultrasound imaging apparatus 300 may display a screen on which first ultrasound images 1010, 1020, and 1030 respectively corresponding to user-designated reference cross-sections 1001 through 1003 are displayed together with a 3D ultrasound image 1040. The ultrasound imaging apparatus 300 displays the first ultrasound images 1010, 1020, and 1030 respectively corresponding to the user-designated reference cross-sections 1001 through 1003 in the 3D ultrasound image 1040 that are set based on a first reference cross-section in with respect to a long axis of the heart. Furthermore, the ultrasound imaging apparatus 300 indicates positions of the user-designated reference cross-sections 1001 through 1003 respectively corresponding to the first ultrasound images 1010, 1020, and 1030.

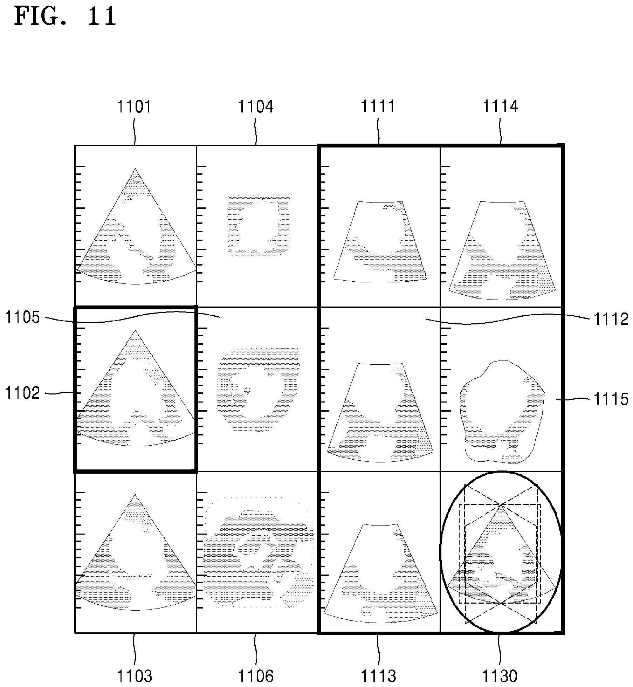

Referring to FIG. 11, the ultrasound imaging apparatus 300 may display at least one first ultrasound image 1111 through 1115 obtained when a 3D ultrasound image 1130 is observed from a view that is different from a 2D ultrasound image based on at least one 2D ultrasound image 1101 through 1106 of the heart. The acquired 2D ultrasound images 1101 through 1106 are cross-sectional images along long and short axes of the heart. The ultrasound imaging apparatus 300 may display a screen including the at least one first ultrasound image 1111 through 1115, the at least one 2D ultrasound image 1101 through 1106, and the 3D ultrasound image 1130.

The ultrasound imaging apparatus 300 may display a reference ultrasound image 1102 to be distinguished from the remaining 2D ultrasound images 1101, 1103, 1104, 1105, and 1106. The reference ultrasound image 1102 corresponds to a reference cross-section used to set a user-designated reference cross-section. A reference ultrasound image may be one of a plurality of 2D ultrasound images.

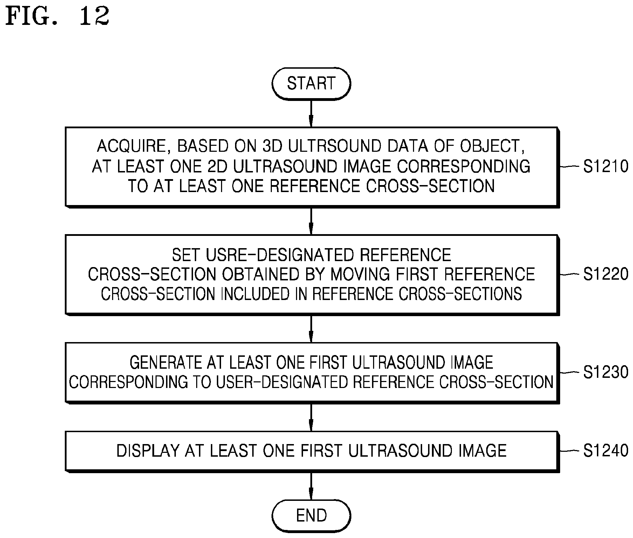

FIG. 12 is a flowchart of a method of operating the ultrasound imaging apparatus 300, according to an exemplary embodiment.

Referring to FIG. 12, the ultrasound imaging apparatus 300 may acquire, based on 3D ultrasound data related to an object, at least one 2D ultrasound image respectively corresponding to at least one reference cross-section (S1210).

The ultrasound imaging apparatus 300 may set in a 3D ultrasound image a user-designated reference cross-section to enable a user to observe a 2D ultrasound image (S1220). The ultrasound imaging apparatus 300 may set a user-designated reference cross-section obtained by moving a first reference cross-section corresponding to a reference ultrasound image selected from among the at least one 2D ultrasound image.

The ultrasound imaging apparatus 300 may receive a first user input for moving the first reference cross-section and set the user-designated reference cross-section based on the received first user input. In this case, the first user input may include at least one of an input for selecting a first reference ultrasound image corresponding to the first reference cross-section from among the at least one 2D ultrasound image and an input of setting information for moving the first reference cross-section. In detail, the setting information may include at least one of pieces of rotation information, tilting information, and vertical movement information that are set with respect to the first reference cross-section.

The ultrasound imaging apparatus 300 may generate at least one first ultrasound image corresponding to the user-designated reference cross-section (S1230). The ultrasound imaging apparatus 300 may set a plurality of user-designated reference cross-sections based on the first reference cross-section. The ultrasound imaging apparatus 300 may generate first ultrasound images respectively corresponding to the plurality of user-designated reference cross-sections.

The ultrasound imaging apparatus 300 may display the generated at least one first ultrasound image (S1240). The ultrasound imaging apparatus 300 may display a screen including a first ultrasound image and a 3D ultrasound image corresponding to a first ultrasound image. In this case, a user-designated reference cross-section corresponding to the first ultrasound image may be displayed to overlap the 3D ultrasound image.

Furthermore, the ultrasound imaging apparatus 300 may display a screen including at least one from among a 3D ultrasound image, at least one 2D ultrasound image, and at least one first ultrasound image. In this case, the at least one 2D ultrasound image may be displayed in such a manner as to be distinguished from the at least one first ultrasound image.

The ultrasound imaging apparatuses described above may be implemented using hardware components, software components, or a combination thereof. For example, the apparatuses and components illustrated in the exemplary embodiments may be implemented using one or more general-purpose or special-purpose computers, such as a processor, a controller, an arithmetic logic unit (ALU), a digital signal processor, a microcomputer, a field programmable array (FPA), a programmable logic unit (PLU), a microprocessor or any other device capable of responding to and executing instructions in a defined manner.

A processing device may run an operating system (OS) and one or more software applications running on the OS. The processing device also may access, store, manipulate, process, and create data in response to execution of software.

Although a single processing device may be illustrated for convenience, one of ordinary skill in the art will appreciate that a processing device may include a plurality of processing elements and/or a plurality of types of processing elements. For example, a processing device may include a plurality of processors or a processor and a controller. In addition, the processing device may have different processing configurations such as parallel processors.

Software may include a computer program, a piece of code, an instruction, or one or more combinations thereof and independently or collectively instruct or configure the processing device to operate as desired.

Software and/or data may be embodied permanently or temporarily in any type of machine, component, physical equipment, virtual equipment, computer storage medium or device, or in a transmitted signal wave so as to be interpreted by the processing device or to provide instructions or data to the processing device. The software also may be distributed over network-coupled computer systems so that the software is stored and executed in a distributed fashion. In particular, the software and data may be stored in one or more computer-readable recording media.

The methods according to the exemplary embodiments may be recorded in non-transitory computer-readable recording media including program instructions to implement various operations embodied by a computer. The non-transitory computer-readable recording media may also include, alone or in combination with the program instructions, data files, data structures, and the like. The program instructions recorded in the non-transitory computer-readable recording media may be designed and configured specially for the exemplary embodiments or be known and available to those of ordinary skill in computer software.

Examples of non-transitory computer-readable recording media include: magnetic media such as hard disks, floppy disks, and magnetic tape; optical media such as CD ROM discs and DVDs; magneto-optical media such as floptical disks; and hardware devices that are specially configured to store and perform program instructions, such as ROM, RAM, flash memory, and the like.

Examples of program instructions include both machine code, such as that produced by a compiler, and higher level code that may be executed by the computer using an interpreter.

The above-described hardware devices may be configured to act as one or more software modules in order to perform the operations of the above-described embodiments, or vice versa.

While one or more exemplary embodiments have been described with reference to the figures, it will be understood by those of ordinary skill in the art that various modifications and changes in form and details may be made from the above descriptions without departing from the scope of the invention as defined by the following claims. For example, adequate effects may be achieved even if the above techniques are performed in a different order than described above, and/or the aforementioned elements, such as systems, structures, devices, or circuits, are combined or coupled in different forms and modes than as described above.

Thus, the scope of the present invention is defined not by the detailed description thereof but by the appended claims.

## Claims

1. An ultrasound imaging apparatus for providing ultrasound images having different viewpoints, the ultrasound imaging apparatus comprising:
an image processor (310) configured to acquire, based on three-dimensional (3D) ultrasound data for generating a 3D ultrasound image of an object, at least one two-dimensional (2D) ultrasound image comprising at least one reference ultrasound image corresponding to at least one reference cross-section;
a controller (320) configured to set a user-designated reference cross-section obtained by moving a first reference cross-section included in the at least one reference cross-section and control generation of at least one first ultrasound image corresponding to the set user-designated reference cross-section; and
a display (330) configured to display the generated at least one first ultrasound image, wherein the user-designated reference cross-section is set to provide the at least one first ultrasound image from a different viewpoint than a viewpoint of the at least one two-dimensional ultrasound image,
wherein the display (330) is configured to display a screen comprising at least one of the at least one 2D ultrasound image and at least one of the at least one first ultrasound image, and wherein the at least one 2D ultrasound image and the at least one first ultrasound image are displayed in such a manner as to be distinguished from each other,
**characterised in that**
the image processor (310) is configured to receive additional 3D ultrasound data related to the object, which is different from the 3D ultrasound data,
wherein the controller (320) is configured to control generation of at least one second ultrasound image corresponding to the user-designated reference cross-section based on the additional 3D ultrasound data,
wherein the display (330) is further configured to display the at least one second ultrasound image, and
wherein the at least one first ultrasound image and the at least one second ultrasound image correspond to the set user-designated reference cross-section.

2. The ultrasound imaging apparatus of claim 1, further comprising a user interface (440) configured to receive a first user input for moving the first reference cross-section,
wherein the controller (420) acquires, based on the received first user input, the user-designated reference cross-section by moving the first reference cross-section.

3. The ultrasound imaging apparatus of claim 2, wherein the first user input comprises at least one of an input for selecting a first reference ultrasound image corresponding to the first reference cross-section from among the at least one 2D ultrasound image and an input of setting information for moving the first reference cross-section.

4. The ultrasound imaging apparatus of claim 3, wherein the setting information comprises at least one of rotation information, tilting information, and vertical movement information that are set with respect to the first reference cross-section.

5. The ultrasound imaging apparatus of claim 1, wherein the object is a heart.

6. The ultrasound imaging apparatus of claim 5, wherein the at least one 2D ultrasound image is a cross-sectional image obtained along at least one of long and short axes of the heart.

7. The ultrasound imaging apparatus of claim 1, wherein the display (330) is further configured to display a
screen comprising at least one of the 3D ultrasound image and the at least one 2D ultrasound image.

8. The ultrasound imaging apparatus of claim 7, wherein the display (330) displays each of the first reference cross-section and the user-designated reference cross-section in such a manner as to overlap the 3D ultrasound image.

9. The ultrasound imaging apparatus of claim 1, wherein the display (330) is configured to display a
screen comprising the 3D ultrasound image, the at least one 2D ultrasound image, and the at least one first ultrasound image.

10. The ultrasound imaging apparatus of claim 1, wherein the display (330) is further configured to display
the 3D ultrasound image including the first reference cross-section and the at least one 2D ultrasound image in such a manner that they are associated with each other, and to display
the 3D ultrasound image including the user-designated reference cross-section and the at least one first ultrasound image in such a manner that they are associated with each other.

11. The ultrasound imaging apparatus of claim 1, wherein the image processor (310) is configured to generate a window to be located on the 3D ultrasound image, to move the window to a position corresponding to the user-designated reference cross-section in the 3D ultrasound image by using setting information of the user-designated reference cross-section, and to acquire the at least one first
ultrasound image at a position corresponding to the user-designated reference cross-section.

12. A method of operating an ultrasound imaging apparatus for providing ultrasound images having different viewpoints, the method comprising:
acquiring (S1210) by means of an image processor (310), based on three-dimensional (3D) ultrasound data for generating a 3D ultrasound image of an object, at least one two-dimensional (2D) ultrasound image comprising at least one reference ultrasound image corresponding to at least one reference cross-section;
setting (S1220) by means of a controller (320) a user-designated reference cross-section obtained by moving a first reference cross-section included in the at least one reference cross-section;
controlling (S 1230) by means of the controller (320) generation of at least one first ultrasound image corresponding to the set user-designated reference cross-section;
displaying (S 1240) the generated at least one first ultrasound image, wherein setting the user-designated reference cross-section provides the at least one first ultrasound image from a different viewpoint than a viewpoint of the at least one two-dimensional ultrasound image, and
wherein the displaying of the generated at least one first ultrasound image (S1240) comprises displaying a screen comprising at least one of the at least one 2D ultrasound image and at least one of the at least one first ultrasound image, and wherein the at least one 2D ultrasound image and the at least one first ultrasound image are displayed in such a manner as to be distinguished from each other.
**characterised in that** the method further comprises:
receiving by means of the image processor (310) additional 3D ultrasound data related to the object, which is different from the 3D ultrasound data,
controlling by means of the controller (320) generation of at least one second ultrasound image corresponding to the user-designated reference cross-section based on the additional 3D ultrasound data, and
displaying the at least one second ultrasound image, and
wherein the at least one first ultrasound image and the at least one second ultrasound image correspond to the set user-designated reference cross-section.

13. The method of claim 12, further comprising receiving a first user input for moving the first reference cross-section,
wherein the setting of the user-designated reference cross-section obtained by moving the first reference cross-section included in the at least one reference cross-section (1220) comprises acquiring, based on the received first user input, the user-designated reference cross-section by moving the first reference cross-section.

## Patentansprüche

1. Ultraschallbildgebungsvorrichtung zum Bereitstellen von Ultraschallbildern mit unterschiedlichen Blickwinkeln, wobei die Ultraschallbildgebungsvorrichtung Folgendes umfasst:
einen Bildprozessor (310), der dazu konfiguriert ist, basierend auf dreidimensionalen (3D) Ultraschalldaten zum Erzeugen eines 3D-Ultraschallbildes eines Objekts mindestens ein zweidimensionales (2D) Ultraschallbild zu erfassen, das mindestens ein mindestens einem Referenz-Querschnitt entsprechendes Referenz-Ultraschallbild umfasst;
eine Steuerung (320), die dazu konfiguriert ist, einen von dem Benutzer festgelegten Referenz-Querschnitt einzustellen, der durch Bewegen eines ersten Referenz-Querschnitts erhalten wird, der in dem mindestens einen Referenz-Querschnitt enthalten ist, und die Erzeugung mindestens eines ersten, dem von dem Benutzer bestimmten Referenz-Querschnitt entsprechenden Ultraschallbildes zu steuern; und
eine Anzeige (330), die dazu konfiguriert ist, das erzeugte mindestens eine erste Ultraschallbild anzuzeigen, wobei der von dem Benutzer festgelegte Referenz-Querschnitt so eingestellt ist, dass er das mindestens eine erste Ultraschallbild aus einem anderen Blickwinkel als einem Blickwinkel des mindestens einen zweidimensionalen Ultraschallbildes bereitstellt,
wobei die Anzeige (330) dazu konfiguriert ist, einen Bildschirm anzuzeigen, der mindestens eines von dem mindestens einen 2D-Ultraschallbild und mindestens eines von dem mindestens einen ersten Ultraschallbild umfasst, und wobei das mindestens eine 2D-Ultraschallbild und das mindestens eine erste Ultraschallbild in einer solchen Weise angezeigt werden, dass sie voneinander verschieden sind,
**dadurch gekennzeichnet, dass** der Bildprozessor (310) dazu konfiguriert ist, zusätzliche 3D-Ultraschalldaten in Bezug auf das Objekt zu empfangen, die sich von den 3D-Ultraschalldaten unterscheiden,
wobei die Steuerung (320) dazu konfiguriert ist, die Erzeugung von mindestens einem zweiten Ultraschallbild, das dem von dem Benutzer festgelegten Referenz-Querschnitt entspricht, basierend auf den zusätzlichen 3D-Ultraschalldaten zu steuern,
wobei die Anzeige (330) ferner dazu konfiguriert ist, das mindestens eine zweite Ultraschallbild anzuzeigen, und
wobei das mindestens eine erste Ultraschallbild und das mindestens eine zweite Ultraschallbild dem von dem Benutzer festgelegten Referenz-Querschnitt entsprechen.

2. Ultraschallbildgebungsvorrichtung nach Anspruch 1, ferner umfassend eine Benutzeroberfläche (440), die dazu konfiguriert ist, eine erste Benutzereingabe zum Bewegen des ersten Referenz-Querschnitts zu empfangen,
wobei die Steuerung (420) basierend auf der empfangenen ersten Benutzereingabe den von dem Benutzer festgelegten Referenz-Querschnitt erfasst, indem sie den ersten Referenz-Querschnitt bewegt.

3. Ultraschallbildgebungsvorrichtung nach Anspruch 2, wobei die erste Benutzereingabe mindestens eine der folgenden Eingaben umfasst: eine Eingabe zum Auswählen eines ersten, dem ersten Referenz-Querschnitt entsprechenden Referenz-Ultraschallbildes aus dem mindestens einen 2D-Ultraschallbild, und eine Eingabe von Einstellungsinformationen zum Bewegen des ersten Referenz-Querschnitts.

4. Ultraschallbildgebungsvorrichtung nach Anspruch 3, wobei die Einstellungsinformationen mindestens eine der folgenden Informationen umfassen: Rotationsinformationen, Neigungsinformationen und Vertikalbewegungsinformationen, die in Bezug auf den ersten Referenz-Querschnitt eingestellt sind.

5. Ultraschallbildgebungsvorrichtung nach Anspruch 1, wobei es sich bei dem Objekt um ein Herz handelt.

6. Ultraschallbildgebungsvorrichtung nach Anspruch 5, wobei es sich bei dem mindestens einen 2D-Ultraschallbild um ein Querschnittsbild handelt, das entlang mindestens einer von der langen und der kurzen Achse des Herzens erhalten wird.

7. Ultraschallbildgebungsvorrichtung nach Anspruch 1, wobei die Anzeige (330) ferner dazu konfiguriert ist, einen Bildschirm anzuzeigen, der mindestens eines von dem 3D-Ultraschallbild und dem mindestens einen 2D-Ultraschallbild umfasst.

8. Ultraschallbildgebungsvorrichtung nach Anspruch 7, wobei die Anzeige (330) sowohl den ersten Referenz-Querschnitt als auch den von dem Benutzer festgelegten Referenz-Querschnitt in einer solchen Weise anzeigt, dass sie das 3D-Ultraschallbild überlappen.

9. Ultraschallbildgebungsvorrichtung nach Anspruch 1, wobei die Anzeige (330) dazu konfiguriert ist, einen Bildschirm anzuzeigen, der das 3D-Ultraschallbild, das mindestens eine 2D-Ultraschallbild und das mindestens eine erste Ultraschallbild umfasst.

10. Ultraschallbildgebungsvorrichtung nach Anspruch 1, wobei die Anzeige (330) ferner dazu konfiguriert ist, das 3D-Ultraschallbild einschließlich des ersten Referenz-Querschnitts und das mindestens eine 2D-Ultraschallbild in einer solchen Weise anzuzeigen, dass sie einander zugeordnet sind, und das 3D-Ultraschallbild einschließlich des von dem Benutzer festgelegten Referenz-Querschnitts und das mindestens eine erste Ultraschallbild in einer solchen Weise anzuzeigen, dass sie einander zugeordnet sind.

11. Ultraschallbildgebungsvorrichtung nach Anspruch 1, wobei der Bildprozessor (310) dazu konfiguriert ist, ein Fenster zu erzeugen, das auf dem 3D-Ultraschallbild angeordnet sein soll, das Fenster zu einer Position zu bewegen, die dem von dem Benutzer festgelegten Referenz-Querschnitt in dem 3D-Ultraschallbild entspricht, indem Einstellungsinformationen des von dem Benutzer festgelegten Referenz-Querschnitts verwendet werden, und das mindestens eine erste Ultraschallbild an einer Position zu erfassen, die dem von dem Benutzer festgelegten Referenz-Querschnitt entspricht.

12. Verfahren zum Betreiben einer Ultraschallbildgebungsvorrichtung zum Bereitstellen von Ultraschallbildern mit unterschiedlichen Blickwinkeln, wobei das Verfahren Folgendes umfasst:
mittels eines Bildprozessors (310), Erfassen (S1210) mindestens eines zweidimensionalen (2D) Ultraschallbildes, das mindestens ein mindestens einem Referenz-Querschnitt entsprechendes Referenz-Ultraschallbild umfasst, und zwar basierend auf dreidimensionalen (3D) Ultraschalldaten zum Erzeugen eines 3D-Ultraschallbildes eines Objekts;
mittels einer Steuerung (320), Einstellen (S1220) eines von dem Benutzer festgelegten Referenz-Querschnitts, der durch Bewegen eines ersten Referenz-Querschnitts erhalten wird, der in dem mindestens einen Referenz-Querschnitt enthalten ist;
mittels der Steuerung (320), Steuern (S1230) der Erzeugung mindestens eines ersten Ultraschallbildes, das dem eingestellten von dem Benutzer festgelegten Referenz-Querschnitt entspricht;
Anzeigen (S1240) des erzeugten mindestens einen ersten Ultraschallbildes,
wobei das Einstellen des von dem Benutzer festgelegten Referenz-Querschnitts das mindestens eine erste Ultraschallbild aus einem anderen Blickwinkel als einem Blickwinkel des mindestens einen zweidimensionalen Ultraschallbildes bereitstellt, und
wobei das Anzeigen des erzeugten mindestens einen ersten Ultraschallbildes (S1240) das Anzeigen eines Bildschirms umfasst, der mindestens eines von dem mindestens einen 2D-Ultraschallbild und mindestens eines von dem mindestens einen ersten Ultraschallbild umfasst, und
wobei das mindestens eine 2D-Ultraschallbild und das mindestens eine erste Ultraschallbild in einer solchen Weise angezeigt werden, dass sie voneinander unterschieden werden können,
**dadurch gekennzeichnet, dass** das Verfahren ferner Folgendes umfasst:
mittels des Bildprozessors (310), Empfangen zusätzlicher 3D-Ultraschalldaten in Bezug auf das Objekt, die sich von den 3D-Ultraschalldaten unterscheiden,
mittels der Steuerung (320), Steuern der Erzeugung mindestens eines zweiten Ultraschallbildes, das dem von dem Benutzer festgelegten Referenz-Querschnitt entspricht, basierend auf den zusätzlichen 3D-Ultraschalldaten, und
Anzeigen des mindestens einen zweiten Ultraschallbildes, und
wobei das mindestens eine erste Ultraschallbild und das mindestens eine zweite Ultraschallbild dem eingestellten, von dem Benutzer festgelegten Referenz-Querschnitt entsprechen.

13. Verfahren nach Anspruch 12, das ferner das Empfangen einer ersten Benutzereingabe zum Bewegen des ersten Referenz-Querschnitts umfasst,
wobei das Einstellen (1220) des von dem Benutzer festgelegten Referenz-Querschnitts, der durch Bewegen des ersten Referenz-Querschnitts erhalten wird, der in dem mindestens einen Referenz-Querschnitt enthalten ist, Folgendes umfasst: Erfassen des von dem Benutzer festgelegten Referenz-Querschnitts durch Bewegen des ersten Referenz-Querschnitts basierend auf der empfangenen ersten Benutzereingabe.

## Revendications

1. Appareil échographique permettant de réaliser des images échographiques présentant différents points de vue, l'appareil échographique comprenant :
un processeur d'image (310) conçu pour acquérir, compte tenu de données échographiques en trois dimensions (3D) permettant de générer une image échographique en 3D d'un objet, au moins une image échographique en deux dimensions (2D) comprenant au moins une image échographique de référence correspondant à au moins une coupe de référence,
un organe de commande (320) conçu pour régler une coupe de référence désignée par l'utilisateur obtenue par déplacement d'une première coupe de référence incluse dans l'au moins une coupe de référence et pour commander la production d'au moins une première image échographique correspondant au réglage de la coupe de référence désignée par l'utilisateur, et
un écran (330) conçu pour afficher l'au moins une première image échographique générée, ladite coupe de référence désignée par l'utilisateur étant réglée pour fournir l'au moins une première image échographique à partir d'un point de vue différent d'un point de vue de l'au moins une image échographique en deux dimensions ;
ledit écran (330) étant conçu pour afficher un visuel comprenant au moins une image parmi l'au moins une image échographique en 2D et au moins une image parmi l'au moins une première image échographique, et ladite au moins une image échographique en 2D et ladite au moins une première image échographique étant affichées de façon distinctive l'une par rapport à l'autre,
**caractérisé en ce que** le processeur d'image (310) est conçu pour recevoir des données échographiques en 3D dites supplémentaires relatives à l'objet, qui sont différentes des données échographiques en 3D,
ledit organe de commande (320) étant conçu pour commander la production d'au moins une deuxième image échographique correspondant à la coupe de référence désignée par l'utilisateur, compte tenu des données échographiques en 3D supplémentaires,
ledit écran (330) étant conçu en outre pour afficher l'au moins une deuxième image échographique, et
ladite au moins une première image échographique et l'au moins une deuxième image échographique correspondent au réglage de la coupe de référence désignée par l'utilisateur.

2. Appareil échographique selon la revendication 1, comprenant en outre une interface utilisateur (440) conçue pour recevoir une première entrée utilisateur visant à déplacer la première coupe de référence,
ledit organe de commande (420) acquérant, compte tenu de la première entrée utilisateur reçue, la coupe de référence désignée par l'utilisateur par déplacement de la première coupe de référence.

3. Appareil échographique selon la revendication 2, dans lequel la première entrée utilisateur comprend une entrée visant à sélectionner une première image échographique de référence correspondant à la première coupe de référence parmi l'au moins une image échographique en 2D et/ou une entrée d'informations de réglage visant à déplacer la première coupe de référence.

4. Appareil échographique selon la revendication 3, dans lequel les informations de réglage comprennent des informations de rotation, des informations d'inclinaison et/ou des informations de mouvement vertical qui sont réglées par rapport à la première coupe de référence.

5. Appareil échographique selon la revendication 1, dans lequel l'objet est un cœur.

6. Appareil échographique selon la revendication 5, dans lequel l'au moins une image échographique en 2D est une image en coupe transversale créée le long d'au moins un axe parmi l'axe long et l'axe court du cœur.

7. Appareil échographique selon la revendication 1, dans lequel l'écran (330) est conçu en outre pour afficher un visuel comprenant l'image échographique en 3D et/ou l'au moins une image échographique en 2D.

8. Appareil échographique selon la revendication 7, dans lequel l'écran (330) affiche la première coupe de référence ainsi que la coupe de référence désignée par l'utilisateur de façon qu'elles chevauchent l'image échographique en 3D.

9. Appareil échographique selon la revendication 1, dans lequel l'écran (330) est conçu pour afficher un visuel comprenant l'image échographique en 3D, l'au moins une image échographique en 2D et l'au moins une première image échographique.

10. Appareil échographique selon la revendication 1, dans lequel l'écran (330) est conçu en outre pour afficher l'image échographique en 3D comportant la première coupe de référence et l'au moins une image échographique en 2D de telle façon qu'elles sont associées l'une à l'autre, et pour afficher l'image échographique en 3D comportant la coupe de référence désignée par l'utilisateur et l'au moins une première image échographique de telle façon qu'elles sont associées l'une à l'autre.

11. Appareil échographique selon la revendication 1, dans lequel le processeur d'image (310) est conçu pour produire une fenêtre destinée à être située sur l'image échographique en 3D, pour déplacer la fenêtre vers une position correspondant à la coupe de référence désignée par l'utilisateur dans l'image échographique en 3D au moyen d'informations de réglage de la coupe de référence désignée par l'utilisateur, et pour acquérir l'au moins une première image échographique en un emplacement correspondant à la coupe de référence désignée par l'utilisateur.

12. Procédé d'exploitation d'un appareil échographique permettant de réaliser des images échographiques présentant différents points de vue, le procédé comprenant :
l'acquisition (S1210), au moyen d'un processeur d'image (310), compte tenu de données échographiques en trois dimensions (3D) permettant de générer une image échographique en 3D d'un objet, d'au moins une image échographique en deux dimensions (2D) comprenant au moins une image échographique de référence correspondant à au moins une coupe de référence,
le réglage (S1220), au moyen d'un organe de commande (320), d'une coupe de référence désignée par l'utilisateur obtenue par déplacement d'une première coupe de référence incluse dans l'au moins une coupe de référence,
la commande (S 1230), au moyen de l'organe de commande (320), de la production d'au moins une première image échographique correspondant au réglage de la coupe de référence désignée par l'utilisateur,
l'affichage (S1240) de l'au moins une première image échographique générée ;
ledit réglage de la coupe de référence désignée par l'utilisateur fournissant l'au moins une première image échographique à partir d'un point de vue différent d'un point de vue de l'au moins une image échographique en deux dimensions, et
ledit affichage de l'au moins une première image échographique générée (S1240) comprenant l'affichage d'un visuel comprenant au moins une image parmi l'au moins une image échographique en 2D et au moins une image parmi l'au moins une première image échographique, et
ladite au moins une image échographique en 2D et ladite au moins une première image échographique étant affichées de façon distinctive l'une par rapport à l'autre,
le procédé étant **caractérisé en ce qu'**il comprend en outre :
la réception, au moyen du processeur d'image (310), de données échographiques en 3D dites supplémentaires relatives à l'objet, qui sont différentes des données échographiques en 3D,
la commande, au moyen de l'organe de commande (320), de la production d'au moins une deuxième image échographique correspondant au réglage de la coupe de référence désignée par l'utilisateur, compte tenu des données échographiques en 3D supplémentaires, et
l'affichage de l'au moins une deuxième image échographique ;
ladite au moins une première image échographique et ladite au moins une deuxième image échographique correspondant à la coupe de référence désignée par l'utilisateur.

13. Procédé selon la revendication 12, comprenant en outre la réception d'une première entrée utilisateur visant à déplacer la première coupe de référence ;
ledit réglage (1220) de la coupe de référence désignée par l'utilisateur obtenue par déplacement de la première coupe de référence incluse dans l'au moins une coupe de référence comprenant l'acquisition, compte tenu de la première entrée utilisateur reçue, de la coupe de référence désignée par l'utilisateur, par déplacement de la première coupe de référence.
